# EUROPEAN PATENT APPLICATION

(11) **EP 4 349 959 A1**
(43) Date of publication of application: **10.04.2024**
(21) Application number: 22811433.6
(22) Date of filing: 27.05.2022
(51) Int. Cl.: C12N 1/12, C12N 5/00

(54) **COMPOSITION FOR CULTURING ALGAE AND ALGAE CULTURING METHOD**

(30) Priority: 28.05.2021 JP 2021090672
(71) Applicant: Tokyo Women's Medical University, Tokyo 162-8666 (JP)
(72) Inventor: SHIMIZU, Tatsuya, Tokyo 162-8666 (JP); HARAGUCHI, Yuji, Tokyo 162-8666 (JP)
(74) Representative: Kransell & Wennborg KB
(86) International application number: PCT/JP2022/021843
(87) International publication number: WO 2022/250165

(57) **Abstract**

The present invention provides an algae culturing method in which a composition containing a medium that has been used to culture animal cells is used as a medium. The present invention further provides a composition for culturing algae, the composition containing a medium that has been used to culture animal cells. Moreover, the present invention provides an algae and animal cell recycle culturing method.

## Description

### FIELD

The present invention relates to a composition for culturing algae and to an algae culturing method. The invention further relates to a recycling culture method for algae and animal cells.

### BACKGROUND

The Food and Agriculture Organization (FAO) predicts a 100% increase in demand for meat during the period from 1999 to 2050 due to global population explosion. Such demand raises concerns regarding worldwide food shortages and rising food costs. Also expected is a global "protein crisis" resulting from a lack of protein supply.

The livestock industry accounts for 18% of greenhouse gas emissions on a global scale, while also increasing emissions in the transportation sector. One solution to food-related problems on the global scale is the use of "cultured foods", as cultured foods are considered to be environmentally-friendly (NPLs 1 and 2). In brief, cultured foods are obtained by *in vitro* culturing of animal cells in medium having a well-defined composition, followed by processing of the animal cells, and they serve as protein-rich foods that can potentially substitute for meat. One of the major advantages of cultured foods is their high efficiency of nutrient production. For example, the protein content of cultured mammalian cells is about 75% (dry weight), which is greater than the protein content of animal muscle tissue (43% dry weight). The use of cultured foods throughout society is expected to substantially reduce the environmental impact of the livestock industry.

The global market for cultured foods is estimated to exceed 600 billion US dollars by 2040. The high demand for cultured foods has led to consumption of huge volumes of medium for large-scale production of animal cells. Each 1 kg of cultured food production requires about 5 × 10¹⁰ cells and 50 L of culture medium (NPL 2). As consumption of cultured foods increases, therefore, this production volume is also expected to increase. Generation of large amounts of waste medium during production of cultured foods is therefore unavoidable. Waste media containing nitrogen and phosphorus-containing nutrients can cause eutrophication of water bodies. The eutrophication due to cultured food production is estimated to be equivalent to eutrophication due to poultry production.

A variety of microalgae exist that grow in freshwater, seawater, or both. Microalgae such as *Chlorococcum* and *Chlorella* are used in the production of petroleum substitutes/biodiesel, bioethanol, fine chemicals, biopharmaceuticals, edible vaccines and hydrogen (NPLs 3 to 9). In addition, *Chlorococcum littorale* which is highly resistant to CO₂ is considered promising for use in effective fixation of CO₂ (NPLs 10 to 12). Research is also actively underway with regard to microalgae and microbial fuel cells that convert light energy to electricity by metabolic reactions of microalgae. Microalgae including *Chlorella vulgaris* are also used in nutritional supplements, pet foods and organic fertilizers. At the current time, Cyanobacteria such as *Arthorospira* and green microalgae such as *Chlorella* are cultured in high volumes for production of health foods (dry weights: 3,000 t and 2,000 t, respectively). Culturing of microalgae requires large volumes of medium containing inorganic salts that include nitrogen and phosphorus-containing compounds. Some reports indicate that microalgae such as *C*. *vulgaris* can prevent eutrophication of water in homes, agriculture and industrial wastewater (NPLs 13 to 15).

Dialysis wastewater is a known type of waste water that is generated by peritoneal dialysis. Peritoneal dialysis is a convenient treatment method with few constraints and numerous advantages, which can be carried out by chronic kidney failure patients themselves with exchange of dialysate in homes or workplaces. A large amount of dialysis wastewater is produced by peritoneal dialysis, making its disposal a significant issue, but a method of using such wastewater for culturing of *Euglena* algae has been reported (PTL 1).

### [CITATION LIST]

### [PATENT LITERATURE]

[PTL 1] Japanese Patent No. 6128510

### [NON PATENT LITERATURE]

[NPL 1] Tuomisto, H. L. & de Mattos, M. J. Environmental impacts of cultured meat production. Environ. Sci. Technol. 45, 6117-6123 (2011).
[NPL 2] Post, M.J. An alternative animal protein source: cultured beef. Ann. N Y Acad. Sci. 1328, 29-33 (2014).
[NPL 3] Ho, S.H. et al. Bioethanol production using carbohydrate-rich microalgae biomass as feedstock. Bioresour. Technol. 135, 191-198 (2013).
[NPL 4] Harun, R. et al. Algal biomass conversion to bioethanol- a step-by-step assessment. Biotechnol. J. 9, 73-86 (2014).
[NPL 5] Norsker, N. H., Barbosa, M. J., Vermue, M. H. & Wijffels, R. H. Microalgal production-a close look at the economics. Biotechnol. Adv. 29, 24-27 (2011).
[NPL 6] Rasala, B. A. et al. Production of therapeutic proteins in algae, analysis of expression of seven human proteins in the chloroplast of Chlamydomonas reinhardtii. Plant Biotechnol. J. 8, 719-733 (2010).
[NPL 7] Gregory, J. A., Topol, A. B., Doerner, D. Z. & Mayfield, S. Alga-produced cholera toxin-Pfs25 fusion proteins as oral vaccines. Appl. Environ. Microbiol. 79, 3917-3925 (2013).
[NPL 8] Specht, E. A. & Mayfield, S. P. Algae-based oral recombinant vaccines. Front. Microbiol. 5, 60 (2014).
[NPL 9] Osanai, T. et al. Pleiotropic effect of sigE over-expression on cell morphology, photosynthesis and hydrogen production in Synechocystis sp. PCC 6803. Plant J. 76, 456-465 (2013).
[NPL 10] Satoh, A., Kurano, N., Harayama, S. & Miyachi, S. Effects of chloramphenicol on photosynthesis, protein profiles and transketolase activity under extremely high CO2 concentration in an extremely-high-CO2-tolerant green microalga, Chlorococcum littorale. Plant Cell Physiol. 45, 1857-1862 (2004).
[NPL 11] Rossi, F., Olguin, E. J., Diels, L. & De Philippis, R. Microbial fixation of CO2 in water bodies and in drylands to combat climate change, soil loss and desertification. N. Biotechnol. 32, 109-120 (2015).
[NPL 12] Cheng, J., Zhu, Y, Zhang, Z. & Yang, W. Modification and improvement of microalgae strains for strengthening CO2 fixation from coal-fired flue gas in power plants. Bioresour. Technol. 291, 121850 (2019).
[NPL 13] Wang, L. et al. Culture of green algae Chlorella sp. in different wastewaters from municipal wastewater treatment plant. Appl. Biochem. Biotechnol. 162, 1174-1186 (2010).
[NPL 14] Chiu, S. Y. et al. Culture of microalgal Chlorella for biomass and lipid production using wastewater as nutrient resource. Bioresour. Technol. 184, 179-189 (2015).
[NPL 15] Wang, J. H. et al. Microalgae-based advanced municipal wastewater treatment for reuse in water bodies. Appl. Microbiol. Biotechnol. 101, 2659-2675 (2017).

### SUMMARY

### [TECHNICAL PROBLEM]

It is an object of the present invention to establish a new method for reducing risk of environmental impact or environmental change by animal cell waste media.

### [SOLUTION TO PROBLEM]

While the use of microalgae has been previously reported for a variety of purposes, the use of microalgae for treatment of media used in animal cell culturing has not been known. The present inventors have conducted much ardent research and have created this invention upon finding that waste media produced by culturing of animal cells can be used for culturing of microalgae. Specifically, the invention encompasses the following.

[1] A method of culturing an algae, using a composition comprising a medium produced by culturing of animal cells as a culture medium.
[2] The method according to [1] above, wherein the animal cells are cells of a vertebrate.
[3] The method according to [1] or [2] above, wherein the animal cells are muscle or liver cells.
[4] The method according to any one of [1] to [3] above, wherein the algae are microalgae.
[5] The method according to any one of [1] to [4] above, wherein the algae are marine and/or euryhaline microalgae.
[6] The method according to any one of [1] to [5] above, wherein the algae are *Chlorococcum littorale* or *Synechococcus sp.*
[7] The method according to any one of [1] to [6] above, wherein the osmotic pressure of the composition is adjusted to an osmotic pressure equivalent to or in a range of ±10% of the osmotic pressure of standard medium for culturing of algae, as necessary.
[8] The method according to any one of [1] to [7] above, wherein an inorganic salt is further added to the composition.
[9] The method according to [8] above, wherein the inorganic salt is one or more selected from the group consisting of sodium salts, potassium salts, magnesium salts and calcium salts.
[10] The method according to any one of [1] to [9] above, wherein the ammonia concentration in the composition is 0.5 mM or greater.
[11] A recycling culture method for algae and animal cells, comprising:
   (1) a step of collecting the algae cultured by the method according to any one of [1] to [10] above,
   (2) a step of decomposing the algae to obtain an algae extract, and
   (3) a step of using the medium containing the algae extract for culturing of animal cells.
[12] The method according to [11] above, wherein the method according to any one of [1] to [10] above is further carried out after step (3).
[13] A composition for culturing of algae comprising a medium produced by culturing of animal cells.
[14] The composition according to [13] above, wherein the animal cells are vertebrate cells.
[15] The composition according to [13] or [14] above, wherein the animal cells are muscle or liver cells.
[16] The composition according to any one of [13] to [15] above, wherein the composition is a composition for culturing of microalgae.
[17] The composition according to any one of [13] to [16] above, wherein the composition is a composition for marine and/or euryhaline microalgae.
[18] The composition according to any one of [13] to [17] above, wherein the composition is used for culturing of *Chlorococcum littorale* or *Synechococcus* sp.
[19] The composition according to any one of [13] to [18] above, wherein the osmotic pressure of the composition is adjusted to an osmotic pressure equivalent to or in a range of ±10% of the osmotic pressure of standard medium for culturing of algae.
[20] The composition according to any one of [13] to [19] above, wherein an inorganic salt is further added.
[21] The composition according to [20] above, wherein the inorganic salt is one or more selected from the group consisting of sodium salts, potassium salts, magnesium salts and calcium salts.
[22] The composition according to any one of [13] to [21] above, wherein the ammonia concentration in the composition is 0.5 mM or greater.

### [ADVANTAGEOUS EFFECTS OF INVENTION]

According to the method of the invention, the waste media from culturing of animal cells, which are produced in large amounts in animal cell culturing steps in the fields of regenerative medicine or biomedicine, or in the field of cultured foods which is expected to widen in coming years, and which have previously been treated as waste, can be utilized for culturing of algae (such as microalgae). It is thus possible to provide a new method whereby animal cell culture is effectively utilized while also contributing to carbon fixation using algae, and reducing environmental impact.

### BRIEF DESCRIPTION OF DRAWINGS

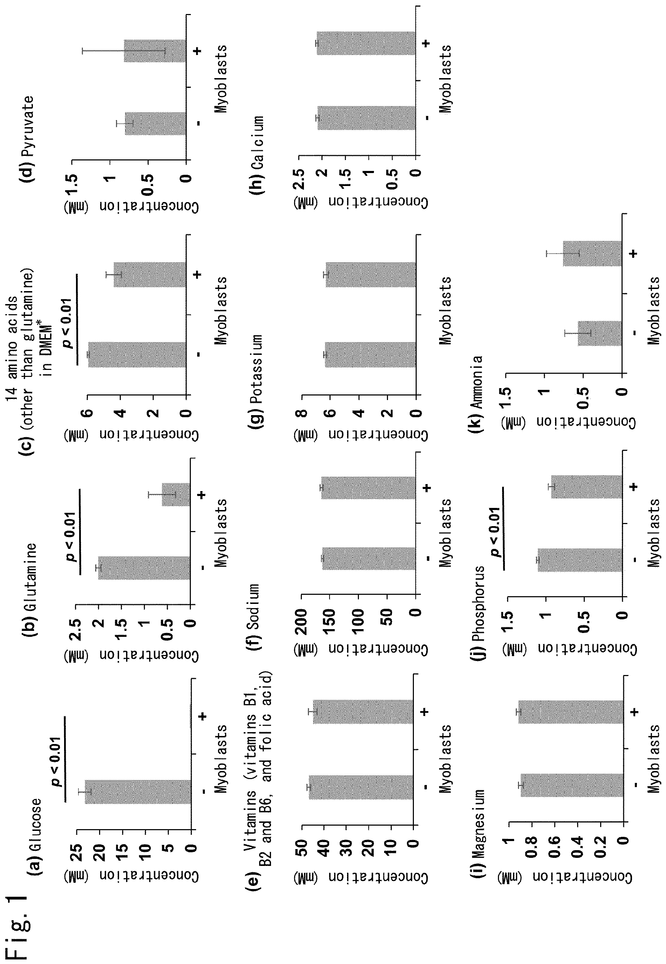

Fig. 1 shows fluctuations in factors in animal cell culture media. Fluctuations in nutrients (a: glucose; b: glutamine; c: total amount of all 14 amino acids in Dulbecco's Modified Eagle's Medium (DMEM) except for glutamine; d: pyruvic acid), vitamins (e: total vitamin B1, B2, B6, folic acid), and inorganic salts (f: sodium; g: potassium; h: calcium; i: magnesium; j: phosphorus; k: ammonia) were analyzed 3 days after culturing using DMEM with addition of 10% fetal bovine serum and 1% penicillin/streptomycin, without (-) and with (+) C2C12 myoblasts. The 14 amino acids in DMEM except for glutamine are: arginine, cysteine, glycine, histidine, isoleucine, leucine, lysine, methionine, phenylalanine, serine, threonine, tryptophan, tyrosine and valine. The data are shown as mean ± SD (n = 4).

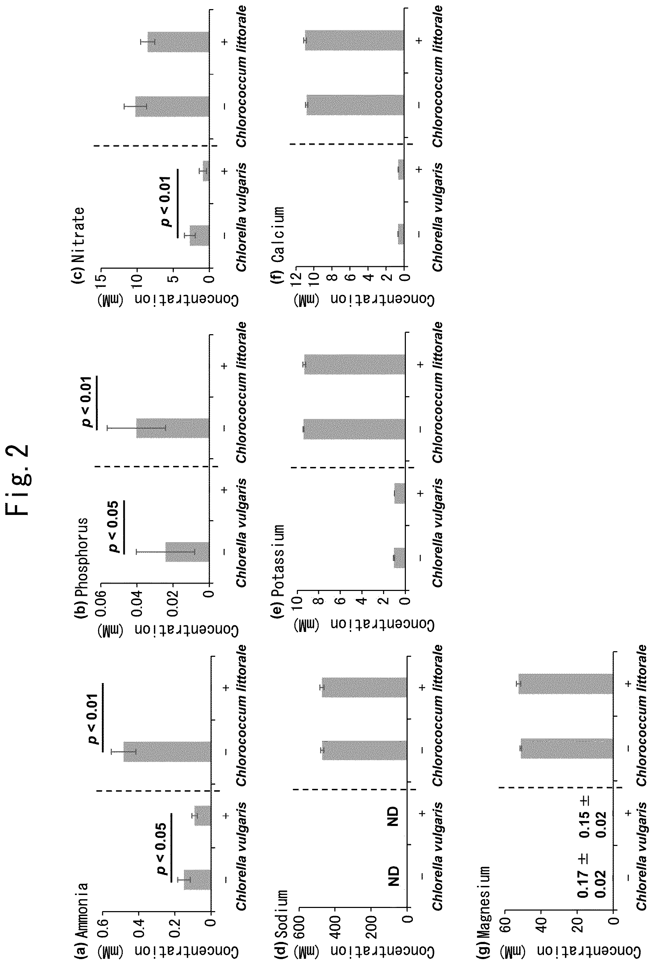

Fig. 2 shows fluctuations in nitrogen- and phosphorus-containing compounds and inorganic salts in medium used for culturing of microalgae. Shown are fluctuations in ammonia (a), phosphorus (b), nitrates (c), sodium (d), potassium (e), calcium (f) and magnesium (g) in standard medium for the microalgae *Chlorella vulgaris* or *Chlorococcum littorale* after culturing for 3 days without (-) or with (+) *Chlorella vulgaris*, or without (-) or with (+) *Chlorococcum littorale.* The data are shown as mean ± SD (n = 4). ND: Not detected.

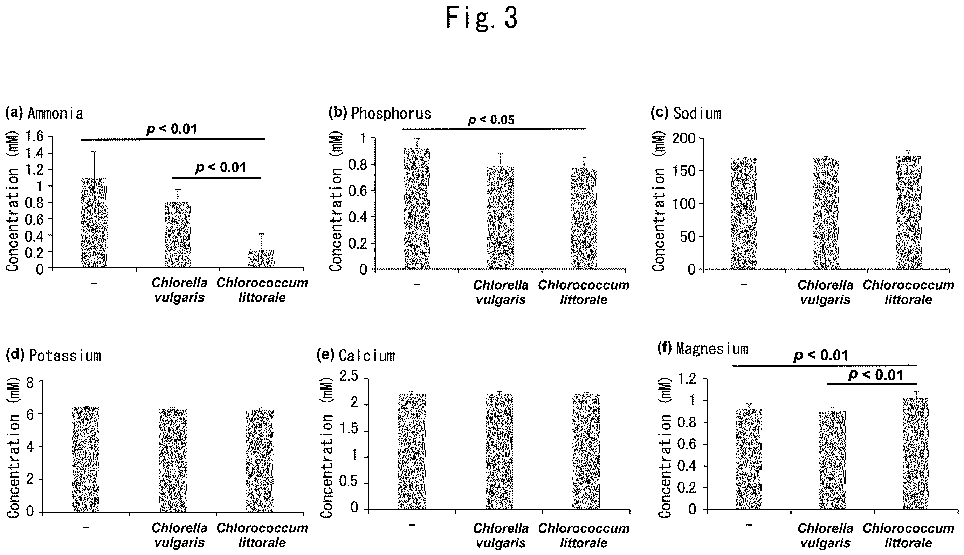

Fig. 3 shows fluctuations in nitrogen- and phosphorus-containing compounds and inorganic salts in medium used for culturing of microalgae. Shown are fluctuations in ammonia (a), phosphorus (b), sodium (c), potassium (d), calcium (e) and magnesium (f) in medium obtained by culturing without microalgae (-) or with microalgae (+) using waste medium produced by culturing C2C12 myoblasts for 3 days. The data are shown as mean ± SD (n = 4).

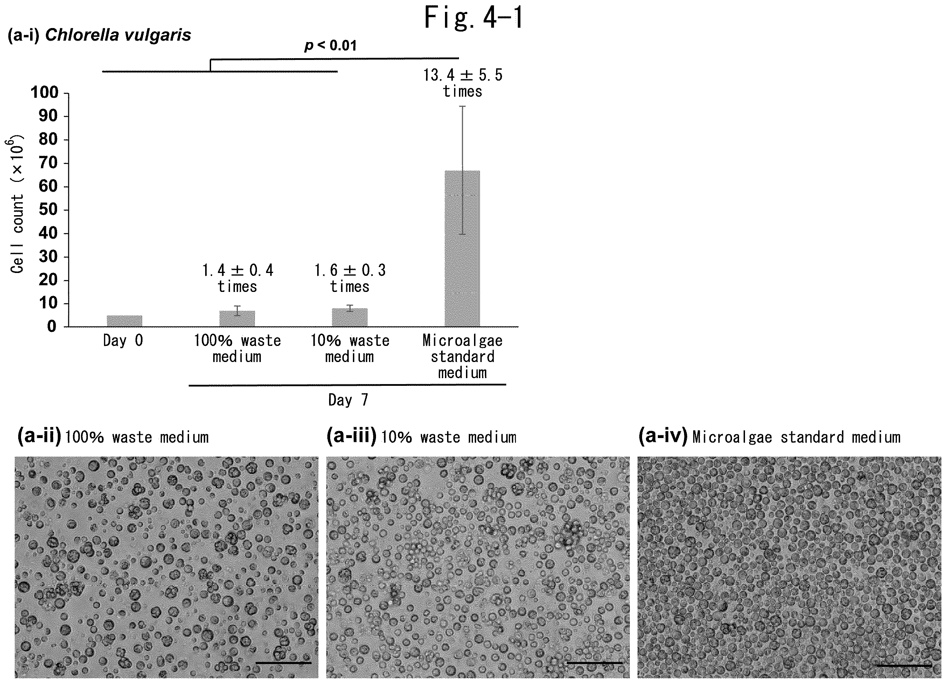

Fig. 4-1 shows cell proliferation of *Chlorella vulgaris* (a-i) in either microalgae standard medium or waste medium from animal cell culture. The *Chlorella vulgaris* were cultured for 7 days either in microalgae standard medium or waste medium from culturing C2C12 myoblasts for 3 days. The data are shown as mean ± SD (n = 4). The waste medium was used without dilution (100% waste medium, photomicrograph: a-ii), or the waste medium was used after 1/10 dilution with purified water (10% waste medium, a-iii). Scale bar: 50 µm.

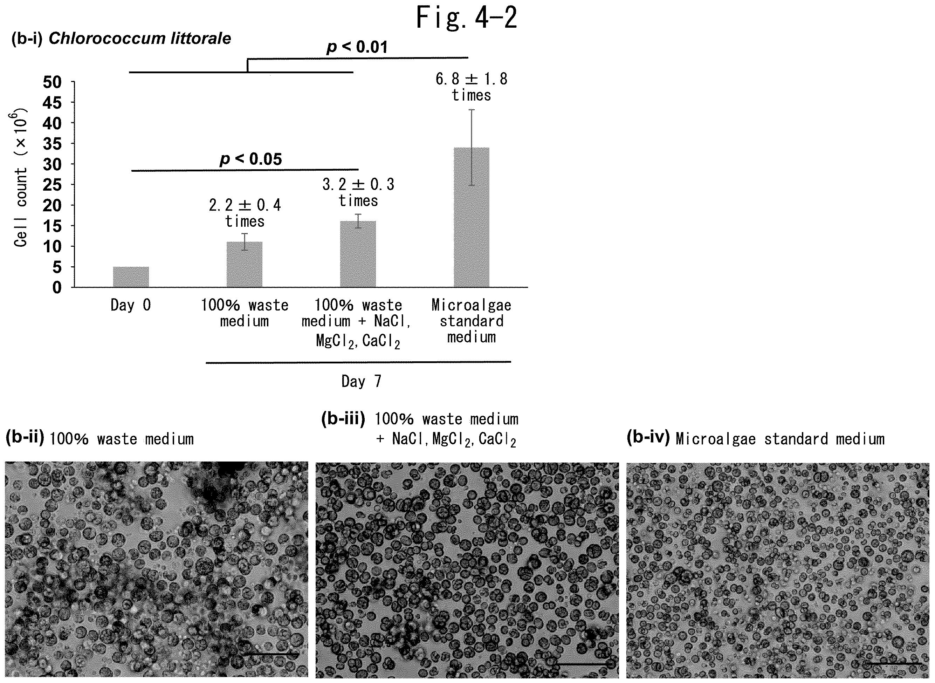

Fig. 4-2 shows cell proliferation of *Chlorococcum littorale* (b-i) in either microalgae standard medium or waste medium from animal cell culture. The *Chlorococcum littorale* were cultured for 7 days either in microalgae standard medium or waste medium from culturing C2C12 myoblasts for 3 days. The data are shown as mean ± SD (n = 4). The medium used was waste medium without addition (100% waste medium, b-ii), waste medium with addition of sodium chloride, magnesium chloride and calcium chloride (100% waste medium + NaCl, MgCl₂, CaCl₂, b-iii), or microalgae standard medium (b-iv). Scale bar: 50 µm.

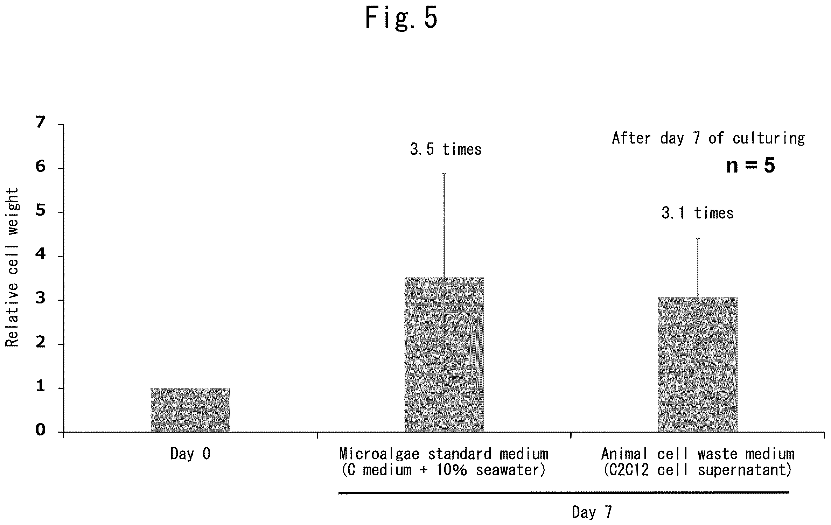

Fig. 5 shows results of culturing algae (*Synechococcus*) using animal cell waste medium.

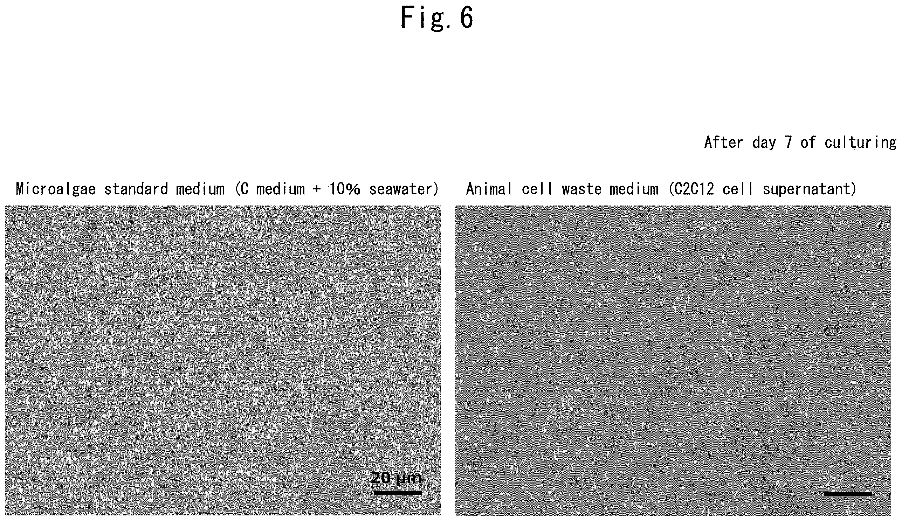

Fig. 6 shows results of culturing algae (*Synechococcus*) using animal cell waste medium (photomicrograph).

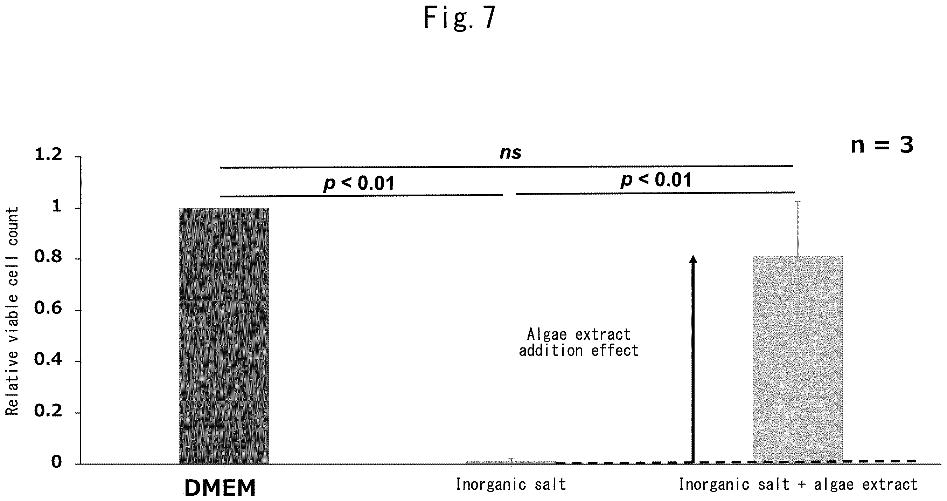

Fig. 7 shows results of culturing animal cells using algae extract obtained from algae cultured using animal cell waste medium.

### DESCRIPTION OF EMBODIMENTS

Embodiments of the invention will now be explained with reference to the accompanying drawings as necessary. The embodiments serve merely for example and the construction of the invention is not limited by the concrete constructions of the embodiments. The prior art documents cited throughout are incorporated herein by reference.

According to one embodiment, the invention provides a composition for culturing of algae comprising a medium produced by culturing of animal cells. According to another embodiment, the invention provides an algae culturing method using a composition comprising a medium produced by culturing of animal cells as a culture medium. According to yet another aspect, the invention provides a recycling culture method for algae and animal cells. According to the invention, media produced by culturing of animal cells, which are produced in large amounts in animal cell culturing steps in the fields of regenerative medicine or biomedicine or in the field of cultured foods, and which have previously been treated as waste, can be utilized for culturing of algae (such as microalgae). This can help contribute to reduction of environmental impact.

The medium produced by culturing of animal cells (also referred to herein as "waste medium" for convenience, while not meaning that it is actual "waste") to be used for the invention, may be a medium that can generally be used for culturing of animal cells, and medium obtained after culturing of animal cells may be used. For use according to the invention, the medium for animal cells before culturing of animal cells is not limited to a publicly known type, but may be Eagle medium, Dulbecco's Modified Eagle Medium (DMEM), DMEM:F12 medium, Glasgow Minimum Essential Medium, Grace's Insect Medium, Ham's Medium, Iscove's Modified Eagle Medium, RPMI-1640 medium, L-15 medium, McCoy's 5AMedium or M199 medium, or any medium that allows culturing of animal cells.

The waste medium to be used for the invention may be any waste medium that has been used for culturing of animal cells. As used herein, "animal cells" includes cells from a vertebrate (mammal (or "mammalian animal"), bird, amphibian, reptile or fish), or cells from an invertebrate (such as a sea squirt, arthropod (crustacean (shrimp or crab, for example) or insect), an echinoderm (such as a sea urchin, sea cucumber or starfish), or a mollusk (such as shellfish, squid or octopus). According to one embodiment, the animal cells may be cells of a vertebrate (for example, a mammal such as a human, monkey, cow, whale, bear, deer horse, pig, boar, sheep, rabbit, rat, mouse, hamster, goat, dog or cat, a bird such as a chicken, domesticated duck, goose, quail, wild duck or pheasant, an amphibian such as a frog, salamander or newt, a reptile such as a crocodile, lizard, snake, tortoise or terrapin, or a fish such as a tuna, salmon, trout, koi fish, shark, eel or puffer fish), an invertebrate (for example, a sea squirt, an arthropod (such as a crustacean (such as shrimp or crab) or an insect), an echinoderm (such as a sea urchin, sea cucumber or starfish), a mollusk (such as a shellfish, squid or octopus), or primary cells, an established cell line or pluripotent stem cells (such as ES cells, ntES cells, Muse cells or iPS cells) or tissue stem cells (such as mesenchymal stem cells) derived from such animals, or cells obtained by inducing differentiation of such cells. The source of the biological tissue for the animal cells may be muscle (such as myoblasts), skin (such as fibroblasts or keratinocytes), liver (such as liver parenchymal cells), kidney (such as renal cells (for example, HEK293)), heart (such as myocardial cells), digestive system (such as oral mucosa cells, intestinal tract epithelial cells or parietal cells), the hematopoietic system (such as hematopoietic stem cells), reproductive tissue (such as ovary cells (for example, CHO cells), sperm cells or uterine epithelial cells), mucosa (such as epithelial cells), or bone tissue (such as osteoblasts or chondrocytes), as well as from any tissues other than these.

The method for culturing the animal cells to be supplied with waste medium to be used for the invention may be any publicly known culturing method (for example, 37°C, saturated steam, humidified atmosphere of 5% CO₂), without any particular restrictions.

Also as used herein, "algae" is a general term referring to organisms that produce oxygen by photosynthesis, with the exclusion of primarily land-inhabiting bryophytes, pteridophytes and spermatophytes. If given an environment necessary for photosynthesis, algae can produce oxygen and nutrients (such as glucose and amino acids) by themselves and proliferate.

The algae to be used for the invention, according to one embodiment, may be "microalgae" (also known as "unicellular algae"). As used herein, the term "microalgae" refers to algae where each consists of a single cell, also including forms with multiple microalgae individuals are gathered together to form microalgae colonies, and which may be freshwater or marine products, or stenohaline or euryhaline microalgae. Examples of microalgae include green algae in which chlorophyll a and b are the major pigments of the chloroplasts, unicellular blue-green algae (cyanobacteria) in which chlorophyll d is the major pigment, and unicellular red algae in which chlorophyll a and phycobilin proteins are the major pigments. More specific examples include the green algae *Chlamydomonas reinhardtii* of the class Chlorophyceae, order Chlamydomonadales (Japanese name: Chlamydomonas) (freshwater), *Dunaliella salina* of the order Dunalielles (Japanese name: Dunaliella) (marine), *Volvox carteri* of the order Volvocales (Japanese name Volvox) (freshwater), *Chlorococcum littorale* of the order Chlorococcales (marine), *Hydrodictyon reticulatum* (Japanese name: Amimidoro) (freshwater), *Pediastrum duplex* (Japanese name: Kunshoumo) (freshwater) and *Scenedesmus dimorphus* (Japanese name: Ikadamo) (freshwater) of the order Sphaeropleales, *Chlorella* sp. of the class Trebouxiophyceae, order Chlorellales (Japanese name: Chlorella) (for example, *Chlorella vulgaris* (freshwater), *Chlorella pyrenoidosa* (freshwater), *Chlorella ellipsoidea* (freshwater) and *Chlorella regularis* (freshwater)), and *Euglena gracilis* and *Euglena proxima* (Japanese name: Midorimushi) (freshwater) of the phylum Euglenozoa, class Euglenophyceae, order Euglena. Unicellular blue-green algae include *Acaryochloris marina* (marine), *Spirulina subsalsa* (freshwater) and *Arthrospira platensis* (freshwater) and *Synechococcus* sp. (marine or freshwater), of the phylum Cyanobacteria. Unicellular red algae include *Cyanidium caldarium* of the phylum Rhodophyta, class Cyanidiophyceae, order Cyanidiales (Japanese name Ideyukogome) (freshwater), and *Galdieria partita* of the phylum Rhodophyta, class Cyanidiophyceae, order Cyanidiales (freshwater). Unicellular charales include *Stichococcus* sp. of the phylum Chlorophyta, class Chlorophyceae, order Klebsormidiales (freshwater). Unicellular ulvophyte algae include filamentous ulvophytes. The algae to be used for the invention are not limited to the algae mentioned above and may also be gene recombinant forms produced by genetic engineering of the aforementioned algae. For example, the algae to be used for the invention may be freshwater or marine and/or stenohaline or euryhaline microalgae, but are preferably marine and/or euryhaline microalgae, such as *Chlorococcum littorale*, or *Synechococcus* sp., although this is not limitative.

The algae to be used for the invention may also be natural algae or algae grown by a publicly known culturing method.

According to one embodiment, the composition comprising the medium produced by culturing of animal cells to be used for culturing of the algae may be standard medium commonly used for culturing of the target algae, prepared as necessary to an osmotic pressure equivalent to or within ±10% (preferably ±5% and more preferably ±3%) of the osmotic pressure of microalgae standard medium, such as AF6 medium, C medium, URO medium or VT medium, for freshwater algae medium, or ESM medium, f/2 medium, IMR medium, MNK medium, C medium + 10% seawater, or a mixture of Daigo IMK or Daigo artificial seawater SP, for marine algae medium. Adjustment of the osmotic pressure may be adjustment by measuring the osmotic pressure according to a publicly known method prior to culturing of the algae, as necessary upon deviation from a range of ±10% (preferably ±5% and more preferably ±3%) of the osmotic pressure of standard medium commonly used for culturing of target algae, for example. The value of the osmotic pressure may be monitored during culturing of the algae, for adjustment as necessary upon deviation from a range of ±10% (preferably ±5% and more preferably ±3%) of the osmotic pressure of standard medium commonly used for culturing of target algae, for example. This will allow the algae proliferation effect to be further augmented. The method of adjusting the osmotic pressure may be any publicly known method, and in cases of high osmotic pressure, adjustment of the osmotic pressure may be by dilution with a hypotonic medium such as water (such as distilled water, ion-exchanged water or sterilized water), or in cases of low osmotic pressure, it may be by addition of a sodium salt (such as sodium chloride, sodium hydroxide or sodium carbonate), a potassium salt (such as potassium chloride, potassium carbonate, potassium hydrogencarbonate or potassium phosphate), a magnesium salt (such as magnesium chloride or magnesium oxide) and/or a calcium salt (such as calcium chloride, calcium carbonate or calcium hydroxide). When the target algae to be cultured is *Chlorococcum littorale*, for example, the osmotic pressure of the obtained composition can be adjusted by adding sodium chloride, magnesium chloride and potassium chloride to the waste medium produced by culturing of animal cells.

According to one embodiment, the composition provided by the invention is a composition for culturing of microalgae, such as a composition for freshwater or marine, or stenohaline or euryhaline microalgae. According to one embodiment, the microalgae to be used for the invention are preferably marine and/or euryhaline microalgae, such as *Chlorococcum littorale* or *Synechococcus* sp., for example.

The culturing conditions for the algae will depend on the type of algae to be cultured, with a temperature of 5 to 40°C, preferably 10 to 35°C and more preferably 10 to 30°C, usually for 1 to 10 days and preferably 3 to 7 days, and by aeration or anaerobic agitation culture, shake culture or stationary culture.

According to one embodiment, the ammonia concentration of the composition to be used for culturing of the algae is 0.5 mM or greater, which allows efficient culturing of algae using ammonia as the nitrogen source. The upper limit for the ammonia concentration of the composition to be used for culturing of the algae is not particularly restricted, but because it contains medium produced by culturing of animal cells, it may be provided at a concentration that allows culturing of animal cells. While not limited, therefore, the ammonia concentration of the composition to be used for culturing of the algae may be 0.5 mM to 10 mM, 0.5 mM to 5 mM or 0.5 mM to 3 mM, for example. Ammonia can serve as a nitrogen source for algae culturing, with additional ammonia optionally being added as nitrogen source as it is consumed by the culturing, and/or with addition of nitrates, either alternatively or additionally.

The invention can also provide a recycling culture method for algae and animal cells. The method may include:
(1) a step of collecting the algae cultured by the method described above,
(2) a step of decomposing the algae to obtain an algae extract, and
(3) a step of culturing animal cells using a medium for culturing of animal cells containing the algae extract.

According to one embodiment, step (2) may be used to obtain an algae extract from algae by carrying out some or all of the steps of the method described in International Patent Publication No. WO2021/066113, for example, and it may include the following substeps, for example:
(2a-1) a step of providing the algae for acid hydrolysis and/or alkali hydrolysis; and
(2a-2) a step of neutralizing the hydrolysate obtained by step (2a-1) to obtain an algae extract.

According to one embodiment, acid hydrolysis alone or alkali hydrolysis alone may be carried out to obtain an algae extract, or both acid hydrolysis and alkali hydrolysis may be carried out. When both acid hydrolysis and alkali hydrolysis are carried out, the alkali hydrolysis may be carried out after acid hydrolysis or the acid hydrolysis may be carried out after alkali hydrolysis. Neutralizing treatment may also be carried out between the acid hydrolysis and alkali hydrolysis.

According to one embodiment, the algae used in step (2a-1) may be supplied for drying treatment before acid hydrolysis and/or alkali hydrolysis.

According to one embodiment, the hydrolysate obtained by step (2-1) may have the hydrolysate neutralized by carrying out the neutralizing step (step (2-2)), to obtain an algae extract to be used for cell culturing. When acid hydrolysis has been carried out at the end of step (2-1), for example, a basic substance or its aqueous solution (sodium hydroxide, potassium hydroxide or an aqueous solution of the same) may be added for neutralization. When alkali hydrolysis has been carried out at the end of step (2-1), an acidic substance (such as sodium hydroxide or potassium hydroxide) or its aqueous solution (such as hydrochloric acid, sulfuric acid, trifluoroacetic acid, *p*-toluenesulfonic acid or methanesulfonic acid) may be added for neutralization.

According to another embodiment, the step of decomposition of the algae of (2) above to obtain an algae extract may also be (2b) a step of decomposition of the algae by ultrasonic waves to obtain an algae extract. The method of decomposition by ultrasonic waves may be any publicly known method, such as the method described in an article by Prabakaran et al. (P. Prabakaran, A.D. Ravindran, A comparative study on effective cell disruption methods for lipid extraction from microalgae. Letters in Applied Microbiology, 53(2) 150-154 (2011)). For example, the algae may be disrupted using a publicly known ultrasonic disruptor (such as a Bioruptor UCD-200TM (Cosmo Bio Co., Ltd., Japan)) at 200 W for 10 minutes on ice to obtain the algae extract.

The step of (2) above may also be a combination of (2b) with steps (2a-1) and (2a-2).

According to one embodiment, step (2) may be carried out under pressure. The term "under pressure" as used herein may be atmospheric pressure, including air pressure conditions higher than 1 atmosphere, such as 1.1 atmospheres or higher, 1.5 atmospheres or higher, 1.8 atmospheres or higher or 2 atmospheres or higher. For example, it may be 1.1 to 300 atmospheres, 1.5 to 200 atmospheres, 1.8 to 100 atmospheres, 2 to 50 atmospheres or 2 to 20 atmospheres. The pressure conditions may be conditions created using any apparatus or method, such as pressurized conditions produced with an autoclave.

### EXAMPLES

The present invention will now be explained in greater detail by Examples, with the understanding that the invention is not limited in any way by the Examples.

### <Example 1>

### 1. Materials and Methods

### 1-1. Culturing of animal myoblasts

C2C12 mouse myoblasts (ATCC^{R} CRL-1772^{™}) were cultured in DMEM (Sigma-Aldrich, St. Louis, MO, USA) supplemented with 10% fetal bovine serum (FBS, Thermo Fisher Scientific, MA, USA) and 1% penicillin/streptomycin (PS, Invitrogen, Carlsbad, CA, USA), at 37°C in a humidified atmosphere containing 5% CO₂³⁷. After seeding 2 × 10⁵ C2C12 cells in a 100 mm culture dish (Greiner Bio-One, Kremsmunster, Austria), they were cultured overnight and the used medium was exchanged with 10 ml of fresh culture medium. After 3 days of culturing, the cell-containing and cell-free media were collected. After centrifugal separation (1,700 × g, 5 min), the harvested culture supernatant (waste medium) was outsourced (SRL, Tokyo, Japan) for further analysis of its constituent factors (Table 1).

**[Table 1]**

| Table 1. Analysis methods | | |
|---|---|---|
| Evaluated factor | Method | References |
| Amino acids | Liquid Chromatography - Mass Spectrometry (LCMS) | Shimbo, K. et al. Biomedical. Chromatogr. 24,683-691 (2010). |
| Ammonia | Colorimetry | Fujii, S. & Okuda, H. Tokushima J. Exp. Med. 22, 1-4(1975). |
| Calcium | Arsenazo III method | Neura, T. Tokyo J. Med. Tech. 34,270-281 (2006) |
| Folic acid | ChemiLuminescence Enzyme Immunoassay (CLEIA) | Nishimura, K. & Mori, K. J. Anal. Bio-Sci. 35, 299-308(2012) |
| Glucose | Hexokinase method | Committee on Biochemical Constituents and Their Analytical Reagents, Japan Society of Clinical Chemistry. Jpn. J. Clin. Chem. 20,247-254 (1991). |
| Magnesium | Xylidyl blue method | Aziz, N.Z. et al. J. Oral. Maxillofac. Pathol. 22,147 (2018). |
| Osmotic pressure | Freezing point depression method | Suzuki, A. & Saito, M. Modern Med. Lab. 6, 759-762(1978). |
| Phosphorus | Direct colorimetry | Drewes, P.A. Clin. Chim. Acta. 39,81-88 (1972). |
| Potassium, sodium | Ion electrode method | Kuwa, K. J. Clin. Lab. Med. 34, 1353-1358(1990); Takahashi K. Modern Med. Lab. 20, 106-110(1992). |
| Pyruvate | Pyruvate oxidase method | Asanuma, K. et al. J. Anal. Bio-Sci. 8,16-24 (1985). |
| Vitamin B 1 | Liquid Chromatography - tandem Mass Spectrometry (LC/MS/MS) | Miyagawa, H. et al. J. Anal. Bio-Sci. 36,327-330 (2013). |
| Vitamin B2, B6 | High-Performance Liquid Chromatography (HPLC) | Yasuda, K. et al. Rinsho Byori. 29,564-568 (1981). |

### 1-2. Microalgae culturing

Freshwater green algae plants C. *vulgaris* (NIES-2170, National Institute for Environmental Studies, Ibaraki, Japan) and seawater green algae plants C. *littorale* (NBRC 102761, National Institute of Technology and Evaluation, Tokyo, Japan) were each cultured in C medium (National Institute for Environmental Studies, Ibaraki, Japan) (reference: "https://mcc.nies.go.jp/medium/ja/c.pdf'; Ichimura, T. 1971 Sexual cell division and conjugation-papilla formation in sexual reproduction of Closterium strigosum. In Proceedings of the Seventh International Seaweed Symposium, University of Tokyo Press, Tokyo, p. 208-214.) or a mixture of Daigo IMK medium (manufacturer: Nihon Pharmaceutical Co., Ltd. (Tokyo, Japan); vendor: FujiFilm-Wako (Osaka, Japan)) and Daigo artificial seawater SP (manufacturer: Nihon Pharmaceutical Co., Ltd. (Tokyo, Japan); vendor: FujiFilm-Wako (Osaka, Japan)), under continuous light (photosynthesis photon flux density, PPFD: 12 ±1 µmol/m²/s, n = 5)^{36,38}. The PPFD was measured using a quantum photometer (Ogawa Seiki Co., Ltd., Tokyo, Japan). After seeding 2 × 10⁸ C. *vulgaris* or 10 × 10⁸ C. *littorale* cells in a 100 mm culture dish (Greiner Bio-One), the cells were cultured for 3 days under continuous light at 30°C, in a humidified atmosphere containing 5% CO₂ (PPFD: 15 ±1 µmol/m²/s, n = 6)³⁹. For the culturing, each microalgae standard medium, or waste medium obtained by culturing C2C12 cells for 3 days, was used after 3 days of culturing with or without microalgae, and supplied for analysis. The microalgae standard medium was medium commonly used for culturing of target algae. This corresponds to, for example, the C medium and the mixture of Daigo IMK medium and Daigo artificial seawater SP. Each medium was collected by centrifugal separation (1,700 × g, 5 min), and the factors in each culture supernatant were analyzed (Table 1). Nitrates were detected using a nitrate meter (LAQUAtwin by As One Corp., Osaka, Japan).

C Medium was used as the microalgae standard medium for C. *vulgaris.* The mixture of Daigo IMK medium and Daigo artificial seawater SP used as the microalgae standard medium for *C*. *littorale* was prepared by dissolving 36 g of Daigo artificial seawater SP (FujiFilm-Wako (Osaka, Japan), product code: 395-01343) and 252 mg of Daigo IMK medium (FujiFilm-Wako (Osaka, Japan), product code: 392-01331) in 1 L of MilliQ water, and treating in an autoclave.

For calculation of the factor consumption (%), the value after culturing of myoblasts/without microalgae was divided by the value after culturing of myoblasts/with microalgae. In order to lower or raise the osmotic pressure of the waste medium after animal cell culturing, there was used purified water (Merck Millipore, Burlington, MA, USA), or sodium chloride (FujiFilm-Wako, Osaka, Japan), magnesium chloride (Fujifilm-Wako Pure Chemical) and calcium chloride (FujiFilm-Wako, Osaka, Japan).

For analysis of cell proliferation, the *C*. *littorale* or *C*. *vulgaris* was seeded at 5 × 10⁶ in a 35 mm culture dish (Greiner Bio-One), and the microalgae were cultured for 7 days at 30°C in a humidified atmosphere containing 5% CO₂, as described above. The medium used for culturing was each microalgae standard medium or waste medium used obtained after 3 days of culturing of C2C12 cells. After culturing, a hemocytometer (Wakenbtech Co., Ltd., Kyoto, Japan) was used to measure the microalgae concentration, and a micropipette (M&S Instruments, Inc., Osaka, Japan) was used to measure the medium volume. The cell count was determined based on the cell concentration and medium volume. An image of the microalgae was recorded under a microscope (ECLIPSE TS2, Nikon, Tokyo, Japan) using NIS-Elements BR software (Nikon).

### 1-3. Statistical analysis

One-way analysis of variance was conducted by unpaired Student's t-test or post-hoc Tukey HSD statistical test for two-group or multi group comparison.

### 2. Results

### 2-1. Culturing of animal myoblasts

After culturing C2C12 myoblasts for 3 days, fluctuation in the factors in the medium was analyzed. The myoblasts actively consumed glucose (99%) (Fig. 1a) and glutamine (69%) (Fig. 1b). However, the amounts of 14 amino acids in basal medium lacking glutamine (Dulbecco's Modified Eagle's Medium, DMEM) only decreased by 26% (Fig. 1c). The amounts of pyruvate (Fig. 1d) and vitamin B 1, B2, B6 and folic acid (Fig. 1e) in the medium were slightly decreased. The amounts of sodium (Fig. 1f), potassium (Fig. 1g), calcium (Fig. 1h) and magnesium (Fig. 1i) in the medium were virtually unchanged. However, the cells had consumed 16% of the phosphorus in the culture medium (Fig. 1j). Since ammonia was produced by decomposition of amino acids, primarily glutamine ²⁷, it was present in the medium even before culturing (data not shown). The ammonia in the medium tended to increase during culturing of the cells (Fig. 1k).

It was thus shown that the levels of nutrients, vitamins and inorganic salts including phosphorus in the culture medium were essentially the same, except for glucose and glutamine which were consumed by the cells and ammonia which was excreted into the waste medium.

### 2-2. Microalgae culturing

Microalgae were cultured for 3 days in normal medium, and fluctuation in factors in the medium was analyzed. The microalgae actively consumed ammonia (*C*. *vulgaris:* 39%; C. *littorale*: 100%) (Fig. 2a), phosphorus (*C*. *vulgaris:* 100%; C. *littorale*: 100%) (Fig. 2b) and nitrates (*C*. *vulgaris:* 67%; *C. littorale*: 17%) (Fig. 2c). However, changes in the amounts of sodium (Fig. 2d), potassium (Fig. 2e), calcium (Fig. 2f) and magnesium (Fig. 2g) were negligible.

Microalgae actively consumed ammonia and phosphorus when proliferated in waste medium from myoblast culture (Fig. 3a and 3b). C. *littorale* ingested more ammonia than *C*. *vulgaris* (*C. littorale*: 80%, *C*. *vulgaris*: 26%). Both microalgae consumed phosphorus (*C*. *littorale*: 16%, *C*. *vulgaris*: 15%). However, the levels of sodium (Fig. 3c), potassium (Fig. 3d) and calcium (Fig. 3e) were virtually unchanged, while the level of magnesium increased with *C*. *littorale* but was virtually unchanged with *C*. *vulgaris* (Fig. 3f).

The present inventors further analyzed whether or not microalgae can proliferate in waste medium from animal cell culture. *C*. *vulgaris* was found to proliferate 13.4-fold in microalgae standard medium, but only proliferated 1.4-fold in waste medium after culturing for 7 days (Fig. 4-1). The levels of inorganic salts in the microalgae standard medium were significantly lower than the levels in the waste medium (Figs. 1 and 2). The waste medium was therefore diluted with water to lower the concentrations. The decrease in osmolarity varied depending on dilution of the medium (Table 2). *C*. *vulgaris* only increased 1.6-fold in the diluted waste medium (Fig. 4- 1). In contrast, the seawater microalgae *C*. *littorale* proliferated 6.8-fold in microalgae standard medium, but proliferated an additional 2.2-fold in waste medium after 7 days of culturing (Fig. 4-2). Since the sodium, magnesium and calcium concentrations in the *C*. *littorale* medium were significantly higher than in the waste medium (Figs. 1 and 2), sodium chloride, magnesium chloride and calcium chloride were added to adjust the concentrations. The final osmotic pressure in the waste medium was roughly equal to the osmotic pressure in the microalgae standard medium (Table 2). Cell proliferation of *C*. *littorale* was augmented 3.2-fold by adjusting the salt concentrations in the waste medium (Fig. 4-2).

**[Table 2]**

| Table 2. Osmotic pressure of waste medium from animal cell culture and microalgae medium | |
|---|---|
| Culture medium | Osmotic pressure (mOsm/KgH₂O) (n = 4) |
| 100% Animal cell culture waste medium | 333 ±6 |
| 10% Animal cell culture waste medium | 35 ±1 |
| 100% Animal cell culture waste medium (containing NaCl, CaCl₂, MgCl₂) | 901 ±8 |
| *Chlorella vulgaris* medium | 13 ±1 |
| *Chlorococcum littorale* medium | 911 ±25 |

### 3. Discussion

It has been reported that freshwater microalgae such as *C*. *vulgaris* can be useful for treatment of home, agricultural and industrial waste water ²⁴⁻²⁶. Conversely, it has been observed that seawater microalgae can effectively treat waste medium after culturing of animal cells (Fig. 3, Fig. 4-1 and Fig. 4-2). In light of differences in the two microalgae in relation to the optimal concentrations of inorganic salts, the present inventors conjecture that microalgae exhibit different preferences for nitrogen-containing compounds. While *C*. *littorale* preferentially consumed ammonia over nitrates, *C*. *vulgaris* preferentially consumed nitrates over ammonia (Figs. 2a and 2c). The difference in ammonia consumption is likely due to the fact that *C*. *littorale* prefers ammonia as a nitrogen source while *C*. *vulgaris* prefers nitrates. Ammonia is the major nitrogen source in cultured cell waste medium, and this is thought to be one reason why *C*. *littorale* proliferated more rapidly than *C*. *vulgaris* in waste medium while proliferation of *C*. *littorale* was slower in waste medium than in normal microalgae standard medium (Fig. 4-2). The waste media after myoblast culturing and the microalgae standard medium both contained ammonia and phosphorus, but only the microalgae standard medium contained nitrates (Figs. 2 and 3). Standard medium for *C*. *littorale* contains significantly more nitrates than ammonia (Figs. 2a and 2c). The amount of ammonia in waste medium may be insufficient with high-density microalgae and long-term culturing of 1 week or longer. Optimization of the medium components and selection of microalgae species, such as microalgae from brackish water²⁸, and microalgae that prefer ammonia or are able to fix nitrogen²⁹, will be a topic for future research. Establishing a culturing system for microalgae using waste media from animal cell cultures will contribute to (1) preserving freshwater and inorganic salts containing ammonia and phosphorus in microalgae cultures, and (2) preventing eutrophication of water caused by treatment of waste media.

Animal myoblasts actively consume glucose and glutamine present in medium (Fig. 1). Glucose is a major carbon source for cell biosynthesis and energy production, and is essential for cell culturing³⁰. Glutamine forms about 5% of amino acid residues in the human proteome³¹. Glutamine is also closely associated with biosynthesis of non-essential amino acids and purine and pyrimidine bases³¹. In proliferating cells, glutamine acts as a biosynthetic precursor for the tricarboxylic acid (TCA) cycle. Here, however, the amounts of nutrients, vitamins and inorganic salts in the culture solution were virtually unchanged (Fig. 1). For estimation of the efficiency of the aforementioned cultured foods, 100% consumption of glucose and amino acids in the medium was assumed. Our research showed, however, about 16% glucose and amino acids, 100% pyruvic acid and 96% vitamins remaining (Fig. 1). In previous research, only 11% glucose and amino acids were consumed by bovine primary muscular tissue cells, whereas 43% pyruvic acid was consumed after 3 days of culturing (data not shown). When metabolism of C2C12 myoblasts was analyzed, the same tendency was observed for levels of glucose and pyruvic acid consumption. The brief explanation is that the cells consumed primarily pyruvic acid after glucose had been consumed (data not shown). This is thought to be the reason for the high deviation in pyruvic acid levels that were found, as shown in Fig. 1d. All of these findings suggest that C2C12 myoblasts prefer glucose over pyruvic acid, in contrast to bovine primary muscular tissue cells that prefer both glucose and pyruvic acid. Since essential nutrients differ significantly by cell type, preparation of medium according to the metabolism of the cell type can provide a more effective cultured food production system. Glucose, pyruvates, amino acids, vitamins and inorganic salts must also be recirculated in waste medium.

While the levels of sodium, potassium and calcium were virtually unchanged in microalgae cultures using myoblast waste media, magnesium increased slightly with *C*. *littorale*, but did not increase with *C*. *vulgaris* (Fig. 3f). It is thought that these differences in data are related to differences in magnesium concentration between the culture media. Since the magnesium concentration in *C*. *littorale* standard medium was significantly higher than the magnesium concentration in *C*. *vulgaris* standard medium or animal cell medium (Figs. 1i and 2g), it may be that magnesium is secreted in low amounts from *C*. *littorale.*

Nutrients and vitamins in mammalian cell culture media are currently derived either directly or indirectly from cereals. The use of cereal-derived nutrients is in competition with food products, and the use of very large amounts of nutrients in cultured foods could potentially lead to increase in cereal costs. Production of cereals for use in chemical fertilizers and agricultural chemicals also consumes high amounts of energy and natural resources, creating environmental stress. The production of ammonia as a major chemical fertilizer is associated with 1 to 2% annual electricity supply on a global scale, 3 to 5% consumption of natural gas on a global scale, and 3% of carbon dioxide emissions on a global scale^{32,33}. Phosphorus, another major chemical fertilizer, is obtained from phosphate rock, but its quality is declining and supply is limited³⁴. Almost all agricultural chemicals are petroleum-based. Microalgae, on the other hand, synthesize nutrients most efficiently, almost without residue³⁵. Animal cell culture systems using nutrients obtained from *C*. *littorale* and *C*. *vulgaris* have been established in recent years, and these sources can efficiently synthesize nutrients necessary for animal cells without competing with food production³⁶. Animal cells can thus proliferate using nutrients produced by microalgae, with the microalgae being able to proliferate by recycling of waste medium from animal cell cultures. Furthermore, *C*. *littorale* is also expected to be efficient for fixing carbon dioxide gas emitted from thermal power plants and steel factories. Recycling culturing systems are envisioned in which animal cells are cultured in nutrients extracted from microalgae grown by absorption of carbon dioxide generated by combustion of fossil fuels, and in waste media generated from cultured foods produced by animal cells. A recycling cell culturing system utilizing animal cells and microalgae could potentially provide solutions for sustainable energy and conservation of nutrients, the environment and resources.

### <Example 2>

*Synechococcus* sp. microalgae (hereunder referred to as "*Synechococcus*"), which are known euryhaline microalgae, were investigated to determine whether or not they can be cultured by waste media obtained after culturing of animal cells.

Waste media obtained from culturing of animal cells used in culturing of *Synechococcus* were prepared by the same method as Example 1.

For analysis of cell proliferation, *Synechococcus* (NIES-3758, National Institute for Environmental Studies) was cultured for 7 days in a 100 mm culture dish (Greiner Bio-One), in C medium + 10% seawater (C+10% seawater, National Institute for Environmental Studies) or waste medium obtained after 3 days of culturing of C2C12 cells, under continuous light (photosynthesis photon flux density PPFD: 15 ±1 µmol/m²/s, n = 6). After collecting the microalgae by centrifugation (2300 × g), the wet weight minus the medium components was measured with a weighing scale (Metler Toledo) to analyze the algae proliferation rate before and after culturing for 7 days. An image of the cultured microalgae was also recorded under a microscope (ECLIPSE TS2, Nikon, Tokyo, Japan) using NIS-Elements BR software (Nikon).

As a result, *Synechococcus* exhibited approximately the same proliferation rate when cultured using waste medium obtained from culturing of animal cells, as when using microalgae standard medium (Fig. 5). Fig. 6 shows the results of microscopic observation after 7 days of culturing, with no change in the microalgae culture density or the form or appearance of the cells whether cultured using waste medium obtained after culturing of animal cells or whether cultured using microalgae standard medium. These results indicated that the euryhaline microalgae *Synechococcus* are able to be cultured and proliferate in waste medium obtained after culturing of animal cells, in a manner similar to microalgae standard medium.

### <Example 3>

When an RL34 mouse hepatocyte line and a bovine muscular tissue cell line were cultured for 3 days under the same conditions as Example 1, and *C*. *littorale* was cultured using each waste medium, culturing was possible in both of the waste media.

### <Example 4>

### Nutrient extraction from algae cultured using animal cell waste medium

Algae (*C*. *littorale*) cultured for 7 days in waste medium from animal muscle cells (C2C12 cells) were acid treated (0.5 N HCl, 100°C, 24 hours) to extract the nutrients. After acid treatment, they were neutralized with NaOH (FujiFilm-Wako, Osaka, Japan), and then the supernatant from centrifugation (12,500 × g, 5 min) was used as the algae extract.

Each nutrient of the algae extract and animal cell basal medium was analyzed and compared by the following methods:
- Glucose analysis: hexokinase method
- Amino acid analysis: liquid chromatography-mass spectrometry (LCMS).

As shown by the following results, nutrients were efficiently extractable even from the algae cultured using animal cell waste medium, and the amounts of glucose and protein constituent amino acids were clearly greater than commercially available basal medium DMEM for animal cell culturing.

**[Table 3]**

| | Glucose concentration (mM) | Total amount of protein constituent amino acids (mM) |
|---|---|---|
| Animal cell waste medium culture algae extract | 78.6 ±19.9 | 25.0 ±3.0 |
| Basal medium DMEM for animal cell culture (high-glucose) | 25 | 10.7 |
| Basal medium DMEM for animal cell culture (low-glucose) | 5.6 | 10.7 |

### <Example 5>

### Culturing of animal cells using nutrients extracted from algae cultured using animal cell waste media (algae extract) (Verification of recycling culture)

The algae extract obtained in Example 4 was used to confirm that culturing of animal muscle cells (C2C12 cells) was possible.

The following three media:
(1) Basal medium DMEM for animal cell culture (Sigma-Aldrich, St. Louis, MO, USA)
(2) Medium containing only inorganic salts in basal medium of (1) (inorganic salt medium)
(3) Inorganic salt medium of (2) + algae extract obtained in Example 3 (5% (v/v))
were used for 2 days of culturing of animal muscle cells (C2C12 cells), and the relative viable cell counts were measured by XTT assay and compared (Fig. 7).

The inorganic salt medium of (2) had the following composition.
(i) Sugar
   · Glucose: 0 mg/L
(ii) Amino acids
   · L-arginine: 0 mg/L
   · L-cysteine: 0 mg/L
   · L-glutamine: 0 mg/L
   · Glycine: 0 mg/L
   · L-histidine: 0 mg/L
   · L-isoleucine: 0 mg/L
   · L-leucine: 0 mg/L
   · L-lysine: 0 mg/L
   · L-methionine: 0 mg/L
   · L-phenylalanine: 0 mg/L
   · L-serine: 0 mg/L
   · L-threonine: 0 mg/L
   · L-tryptophan: 0 mg/L
   · L-tyrosine: 0 mg/L
   · L-valine: 0 mg/L
(iii) Vitamins
   · Pantothenic acid: 0 mg/L
   · Choline chloride: 0 mg/L
   · Folic acid: 0 mg/L
   · i-Inositol: 0 mg/L
   · Niacinamide: 0 mg/L
   · Pyridoxine: 0 mg/L
   · Riboflavin: 0 mg/L
   · Thiamine: 0 mg/L
(iv) Minerals
   · CaCl₂: 200 mg/L
   · KCl: 400 mg/L
   · Fe(NO₃)₃·9H₂O: 0.10 mg/L
   · MgSO₄: 98 mg/L
   · NaCl: 6400 mg/L
   · NaHCO₃: 3700 mg/L
   · NaH₂PO₄: 109 mg/L
   · Phenol red: 15 mg/L

The results indicated that animal muscle cells can be cultured using algae extract obtained from algae proliferated in animal muscle cell waste medium, confirming that it is possible to established a recycling culture system with algae and animal cells.

### References:

1. FAO. World Livestock 2011 -Livestock in Food Security (FAO, 2011).
2. Tuomisto, H. L. & de Mattos, M. J. Environmental impacts of cultured meat production. Environ. Sci. Technol. 45, 6117-6123 (2011).
3. Post, M.J. An alternative animal protein source: cultured beef. Ann. N Y Acad. Sci. 1328, 29-33 (2014).
4. Lodish, H. et al. Molecular Cell Biology, 3rd edition, Ch. 5 (W. H. Freeman and Company, 1995).
5. Spolaore, P., Joannis-Cassan, C., Duran, E. & Isambert, A. Commercial applications of microalgae. J. Biosci. Bioeng. 101, 87-96 (2006).
6. Gerhardt, C. et al. How will cultured meat and meat alternatives disrupt the agricultural and food industry? Ind. Biotechnol. 16, 2020 (2020).
7. Mattick, C. S., Landis, A. E., Allenby, B. R. & Genovese, N. J. Anticipatory life cycle analysis of in vitro biomass culture for cultured meat production in the United States. Environ. Sci. Technol. 49, 11941-11949 (2015).
8. Chisti, Y. Biodiesel from microalgae. Biotechnol. Adv. 25, 294-306 (2007).
9. Ajjawi, I. et al. Lipid production in Nannochloropsis gaditana is doubled by decreasing expression of a single transcriptional regulator. Nat. Biotechnol. 35, 647-652 (2017).
10. Ho, S.H. et al. Bioethanol production using carbohydrate-rich microalgae biomass as feedstock. Bioresour. Technol. 135, 191-198 (2013).
11. Harun, R. et al. Algal biomass conversion to bioethanol - a step-by-step assessment. Biotechnol. J. 9, 73-86 (2014).
12. Norsker, N. H., Barbosa, M. J., Vermue, M. H. & Wijffels, R. H. Microalgal production-a close look at the economics. Biotechnol. Adv. 29, 24-27 (2011).
13. Rasala, B. A. et al. Production of therapeutic proteins in algae, analysis of expression of seven human proteins in the chloroplast of Chlamydomonas reinhardtii. Plant Biotechnol. J. 8, 719-733 (2010).
14. Gregory, J. A., Topol, A. B., Doerner, D. Z. & Mayfield, S. Alga-produced cholera toxin-Pfs25 fusion proteins as oral vaccines. Appl. Environ. Microbiol. 79, 3917-3925 (2013).
15. Specht, E. A. & Mayfield, S. P. Algae-based oral recombinant vaccines. Front. Microbiol. 5, 60 (2014).
16. Osanai, T. et al. Pleiotropic effect of sigE over-expression on cell morphology, photosynthesis and hydrogen production in Synechocystis sp. PCC 6803. Plant J. 76, 456-465 (2013).
17. Satoh, A., Kurano, N., Harayama, S. & Miyachi, S. Effects of chloramphenicol on photosynthesis, protein profiles and transketolase activity under extremely high CO2 concentration in an extremely-high-CO2-tolerant green microalga, Chlorococcum littorale. Plant Cell Physiol. 45, 1857-1862 (2004).
18. Rossi, F., Olguin, E. J., Diels, L. & De Philippis, R. Microbial fixation of CO2 in water bodies and in drylands to combat climate change, soil loss and desertification. N. Biotechnol. 32, 109-120 (2015).
19. Cheng, J., Zhu, Y, Zhang, Z. & Yang, W. Modification and improvement of microalgae strains for strengthening CO2 fixation from coal-fired flue gas in power plants. Bioresour. Technol. 291, 121850 (2019).
20. Lee, D. J., Chang, J. S. & Lai, J. Y. Microalgae-microbial fuel cell: A mini review. Bioresour. Technol. 198, 891-895 (2015).
21. McCusker, S., Buff, P. R., Yu, Z. & Fascetti, A. J. Amino acid content of selected plant, algae and insect species: a search for alternative protein sources for use in pet foods. J. Nutr. Sci. 3, e39 (2014).
22. Gutierrez-Salmean, G., Fabila-Castillo, L. & Chamorro-Cevallos, G. Nutritional and toxicological aspects of Spirulina (Arthrospira). Nutr. Hosp. 32, 34-40 (2015).
23. Alori, E. T. & Babalola, O. O. Microbial inoculants for improving crop quality and human health in Africa. Front. Microbiol. 9, 2213 (2018).
24. Wang, L. et al. Culture of green algae Chlorella sp. in different wastewaters from municipal wastewater treatment plant. Appl. Biochem. Biotechnol. 162, 1174-1186 (2010).
25. Chiu, S. Y. et al. Culture of microalgal Chlorella for biomass and lipid production using wastewater as nutrient resource. Bioresour. Technol. 184, 179-189 (2015).
26. Wang, J. H. et al. Microalgae-based advanced municipal wastewater treatment for reuse in water bodies. Appl. Microbiol. Biotechnol. 101, 2659-2675 (2017).
27. Schneider, M., Marison, I. W. & von Stockar, U. The importance of ammonia in mammalian cell culture. J. Biotechnol. 46, 161-185 (1996).
28. Tanabe, Y, Hodoki, Y, Sano, T., Tada, K. & Watanabe, M. M. Adaptation of the freshwater bloom-forming cyanobacterium Microcystis aerguinosa to brackish water is driven by recent horizontal transfer of sucrose genes. Front. Microb. 9, 1150 (2018).
29. Tsujimoto, R. et al. Functional expression of an oxygen-labile nitrogenase in an oxygenic photosynthetic organism. Sci. Rep. 8, 7380 (2018).
30. Mulukutla, B. C., Yongky, A., Le, T., Mashek, D. G. & Hu, W. S. Regulation of glucose metabolism - A perspective from cell bioprocessing. Trends Biotechnol. 34, 638-651 (2016).
31. Zhang, J., Pavlova, N. N. & Thompson, C. B. Cancer cell metabolism: the essential role of the nonessential amino acid, glutamine. EMBO J. 36, 1302-1315 (2017).
32. Ye, Y et al. A critical review on ammonium recovery from wastewater for sustainable wastewater management. Bioresour. Technol. 268, 749-758 (2018).
33. Song, Y et al. A physical catalyst for the electrolysis of nitrogen to ammonia. Sci. Adv. 4, e1700336 (2018).
34. Tarayre, C. et al. New perspectives for the design of sustainable bioprocesses for phosphorus recovery from waste. Bioresour. Technol. 206, 264-274 (2016).
35. Singh, A. K. & Singh, M. P. Importance of algae as a potential source of biofuel. Cell Mol. Biol. 60, 106-109 (2014).
36. Okamoto, Y, Haraguchi, Y, Sawamura, N., Asahi, T. & Shimizu, T. Mammalian cell culture using nutrients extracted from microalgae. Biotechnol. Prog. 36, e2941 (2020).
37. Haraguchi, Y, et al. Fabrication of functional three-dimensional tissues by stacking cell sheets in vitro. Nat. Protoc. 7, 850-858 (2012).
38. Haraguchi, Y, et al. Thicker three-dimensional tissue from a "symbiotic recycling system" combining mammalian cells and algae. Sci. Rep. 7, 41594 (2017).
39. Haraguchi, Y & Shimizu, T. Three-dimensional tissue fabrication system by co-culture of microalgae and animal cells for production of thicker and healthy cultured food. Biotechnol. Lett. in press. doi: 10.1007/s10529-021-03106-0.

## Claims

1. A method of culturing an algae, using a composition comprising a medium produced by culturing of animal cells as a culture medium.

2. The method according to claim 1, wherein the algae are microalgae.

3. The method according to claim 1 or 2, wherein the algae are marine and/or euryhaline microalgae.

4. The method according to any one of claims 1 to 3, wherein the osmotic pressure of the composition is adjusted to an osmotic pressure equivalent to or in a range of ±10% of the osmotic pressure of standard medium for culturing of algae, as necessary.

5. The method according to any one of claims 1 to 4, wherein an inorganic salt is further added to the composition.

6. The method according to claim 5, wherein the inorganic salt is one or more selected from the group consisting of sodium salts, potassium salts, magnesium salts and calcium salts.

7. The method according to any one of claims 1 to 6, wherein the ammonia concentration in the composition is 0.5 mM or greater.

8. A recycling culture method for algae and animal cells, comprising:
(1) a step of collecting the algae cultured by the method according to any one of claims 1 to 7,
(2) a step of decomposing the algae to obtain an algae extract, and
(3) a step of culturing animal cells using a medium containing the algae extract.

9. The method according to claim 8, wherein the method according to any one of claims 1 to 7 is further carried out after step (3).

10. A composition for culturing of algae comprising a medium produced by culturing of animal cells.

11. The composition according to claim 10, wherein the composition is a composition for culturing of microalgae.

12. The composition according to claim 10 or 11, wherein the composition is a composition for marine and/or euryhaline microalgae.

13. The composition according to any one of claims 10 to 12, wherein the osmotic pressure of the composition is adjusted to an osmotic pressure equivalent to or in a range of ±10% of the osmotic pressure of standard medium for culturing of algae.

14. The composition according to any one of claims 10 to 13, wherein an inorganic salt is further added.

15. The composition according to claim 14, wherein the inorganic salt is one or more selected from the group consisting of sodium salts, potassium salts, magnesium salts and calcium salts.

16. The composition according to any one of claims 10 to 15, wherein the ammonia concentration in the composition is 0.5 mM or greater.
